# EUROPEAN PATENT SPECIFICATION

(11) **EP 3 936 168 B1**
(45) Date of publication and mention of the grant of the patent: **19.02.2025**
(21) Application number: 20185230.8
(22) Date of filing: 10.07.2020
(51) Int. Cl.: A61M 1/00

(54) **A SUCTION CONTROL HANDLE COMPRISING A COVER ELEMENT**
SAUGSTEUERGRIFF MIT EINEM ABDECKELEMENT
POIGNÉE DE COMMANDE D'ASPIRATION COMPRENANT UN ÉLÉMENT DE COUVERCLE

(43) Date of publication of application: 12.01.2022
(73) Proprietor: Karlsmose, Bo, 8270 Højbjerg (DK); Lüscher, Michael, 8230 Åbyhøj (DK); Karlsmose, Per, 2100 Copenhagen Ø (DK)
(72) Inventor: Karlsmose, Bo, 8270 Højbjerg (DK); Lüscher, Michael, 8230 Åbyhøj (DK); Karlsmose, Per, 2100 Copenhagen Ø (DK)
(74) Representative: Høiberg P/S

(56) References cited:
- US-A- 3 625 221
- US-A1- 2004 230 169
- US-A1- 2005 148 937
- US-B2- 7 802 574

## Description

The present disclosure relates to medical suction devices; in particular a suction control handle comprising a cover element.

### Background of invention

Medical suction devices are used in many applications within the healthcare system for removing unwanted substances (e.g. blood, secretion, mucus, tissue, ear wax, pus, other body fluids, or liquids e.g. saltwater, medicine, or chemicals) from a patient by suction. Furthermore, medical suction devices are used for similar purposes by veterinarians for treating animals. The medical suction devices generate a vacuum (or is connected to a vacuum supply), such that the unwanted substance(s) may be removed by suction. This procedure is sometimes also referred to as micro suction. In some procedures involving medical suction devices, the operator of the device (e.g. a doctor or a medical assistant), can regulate the airflow through the device by covering a small aeration hole with his/her finger. This aeration hole is often placed in a suction control handle constituting a gripping part for the operator, whereby the airflow (and thereby the vacuum force) can be regulated using a finger such as the thumb of the operator. If the aeration hole is completely covered, the vacuum force is strong and if the hole is not covered, the vacuum force is weaker. If partially covered, the vacuum force is somewhat in between these two extremes. In this way, the operator can quickly and easily adjust the suction force by covering the aeration hole with his finger, in order to remove unwanted substances in sensitive areas of the psychical body (e.g. ears, nose, throat, eyes, lungs, uro-genital, internal organs etc.).

In the present procedures using such medical suction devices, the operator's vacuum regulating finger is at risk of exposure to contaminants from the patient, such as microorganisms (e.g. bacteria, virus, parasites, fungus), chemicals and/or other contaminants. The operator normally switches her/his hands to other devices during the procedure, thereby introducing a risk of spreading contamination from the patient to a variety of other devices and/or surfaces unless the operator removes her/his gloves or washes her/his hands during the procedure, thereby stopping the chain of infection. In the current modus operandi, the operator typically carries out the procedure single-handedly, and oftentimes the operator does not change the gloves in between operating the different devices required in the procedure.

The medical suction devices have different designs depending on the intended use.

However, they often employ a suction control handle for regulating the airflow / vacuum force, and the control handle can similarly have various designs depending on the application. Some control handles are configured to connect a suction tube at the proximal end of the control handle and a suction pipe at the distal end of the control handle. Oftentimes, the suction pipe is intended for single-use, i.e. it is discarded after use on one patient, whereas the control handle itself might be used several times throughout the day, i.e. for treating a plurality of patients. This adds to the problem outlined above, since contaminants from many patients may accumulate in/near the aeration hole of the suction control handle, which is frequently touched by the operator of the suction device.

US 3,625,221 relates to a control valve for adjustably controlling the suction in a catheter having an elongated suction tube provided with a portion defining an aperture for admitting air to the tube to vary the suction action. The suction control comprises an aperture closing means herein defined by a resilient member having a first portion carried on the tube adjacent the aperture and a second portion yieldingly spaced adjacent the aperture and resiliently manually deflectable progressively across the aperture.

Summarizing, there is a need of a suction control handle for a medical suction device, wherein the suction control handle can be operated with a reduced risk of contamination from the patient. Ideally, the control handle eliminates the risk of contamination when operating the control handle, and ideally the solution is compatible with existing suction control handles.

### Summary of invention

A suction control handle according to the present invention comprises the technical features as defined in independent claim 1.

The present disclosure addresses the above-mentioned needs by providing a suction control handle comprising a cover element for covering the aeration hole during use and for protecting the operator from contamination from any possible contaminants (e.g. bacteria, virus, parasites, fungus) exiting through said aeration hole during a treatment procedure.

Accordingly, the present disclosure relates to a suction control handle for a medical suction device, said medical suction device being configured for creating a vacuum or connected to a vacuum supply, such that an airflow is present through the suction control handle during use, the suction control handle comprising an aeration hole for regulating the airflow through the suction control handle during use; and a cover element attached to the suction control handle, and formed such that, in use, the airflow through the suction control handle can be gradually adjusted by covering the aeration hole, wholly or partly, by the cover element. Preferably, the suction control handle can be operated by a single hand such that the cover element may be operated by a finger, e.g. the thumb of the person operating the suction control handle.

The present disclosure further relates to a method (not claimed) of adjusting the vacuum force of a medical suction device, said method comprising the steps of: providing a suction control handle as disclosed herein, wherein the suction control handle forms part of the medical suction device, said medical suction device being connected to a vacuum supply; running the vacuum supply in order to provide an airflow through the suction control handle; and covering the aeration hole, wholly or partly, by the cover element of the suction control handle, such that the airflow is gradually adjusted, thereby adjusting the vacuum force of the medical suction device.

### Description of drawings

Fig. 1 shows an embodiment of the suction control handle comprising a cover element, wherein the cover element is a bendable flap attached to the handle.
Fig. 2 shows an embodiment of the suction control handle comprising a cover element, wherein the cover element is a bendable flap comprising a pocket for receiving a finger.
Fig. 3 shows an embodiment of the suction control handle comprising a cover element, wherein the cover element is a bendable flap comprising a second protrusion suitable for insertion in the aeration hole of the control handle.
Fig. 4 shows an embodiment of the suction control handle, wherein the proximal end of the suction control handle is configured to connect to a first suction tube, and the distal end of the suction control handle is configured to connect to a second suction tube.
Fig. 5 shows an embodiment of the suction control handle, wherein the cover element comprises an elastic band configured for securing the cover element to the suction control handle.
Fig. 6 shows an embodiment of the suction control handle comprising a cover element, wherein the free end of the cover element is curved backwards to form a pocket.
Fig. 7 shows an embodiment of a cover element according to the present disclosure. In this embodiment, the cover element comprises a first protrusion configured to mechanically engage with a groove in the suction control handle.
Fig. 8 shows an embodiment of the suction control handle, wherein the handle comprises a groove configured to receive and secure the first protrusion of the cover element such that the cover element can be removably attached to the control handle by sliding the first protrusion into said groove.
Fig. 9 shows the embodiment of Fig. 8, wherein the first protrusion of the cover element has been fully inserted in the groove, such that the fixed end of the cover element is fixed to the suction control handle by the groove.
Fig. 10 shows how a cover element comprising an elastic band may be removably attached to the suction control handle by mounting the elastic band around the control handle.
Fig. 11 shows a side view of an embodiment of a suction control handle, said control handle comprising a slidable cover plate configured for covering the aeration hole.
Fig. 12 shows a perspective view of the embodiment shown in Fig. 11.

### Detailed description of the invention

The present disclosure relates to a suction control handle comprising a cover element.

The suction control handle is configured for use with a medical suction device for removing unwanted substances (e.g. blood, secretion, mucus, tissue, ear wax, pus, other body fluids, or liquids e.g. saltwater, medicine, or chemicals) from a patient. The substances may typically be in the form of droplets, aerosols, tissue, etc. In many hospitals and other health facilities, suction is typically provided by suction regulators (also referred to herein as suction control handles), connected to a central medical vacuum supply by way of a pipeline system. In the present context, a medical suction device should be understood as a device for providing suction e.g. through a tube, regardless of the position of the vacuum source. Hence, either the medical suction device is configured to create a vacuum or it is connected to an external vacuum supply. A medical suction device will typically comprise a suction control handle for adjusting the vacuum force of the suction device, and one or more tubes or pipes connected to the control handle, wherein the suction device is connected to a vacuum supply. An example of a medical suction device is an ENT (Ear, Nose, & Throat) suction device.

The presently disclosed suction control handle comprises a channel extending through the control handle, wherein an airflow is present in said channel during use of the medical suction device, and wherein the unwanted substances may be transported in the direction of said airflow. The suction control handle is preferably adapted to be held by one hand during suction, and configured such that an operator holding the handle can regulate the vacuum force of the medical suction device. The suction control handle comprises at least one aeration hole fluidly connected to aforementioned channel. The aeration hole is to be understood as an opening in the suction control handle, said opening fluidly connecting the channel inside the control handle with the outside environment that the control handle is situated in.

Existing suction control handles typically allow the operator to regulate the vacuum force by covering the aeration hole with a finger, whereby the speed of the airflow in the channel is increased and consequently the vacuum force is increased. Hence, preferably the aeration hole has a size that may be covered using a single finger, e.g. the thumb of an operator handling the device. The presently disclosed suction control handle comprises a cover element attached to the suction control handle, and formed such that, in use, the airflow through the suction control handle can be gradually adjusted by covering the aeration hole, wholly or partly, by the cover element. Hence, the cover element has a sufficient size to cover the aeration hole. Thereby, the operator's finger is not in direct contact with the aeration hole. Consequently, the cover element protects the operator from contaminants possibly exiting through said aeration hole. A further advantage of the presently disclosed suction control handle, is that the operator may gradually adjust the airflow / vacuum force.

The suction control handle may come in a variety of embodiments. According to one embodiment, the proximal end of the suction control handle is configured to connect to a suction tube, e.g. a flexible suction tube, and the distal end of the suction control handle is configured to connect to a suction pipe, e.g. a rigid suction pipe. The suction pipe is preferably suitable for removing unwanted substances by suction when it forms part of the medical suction device.

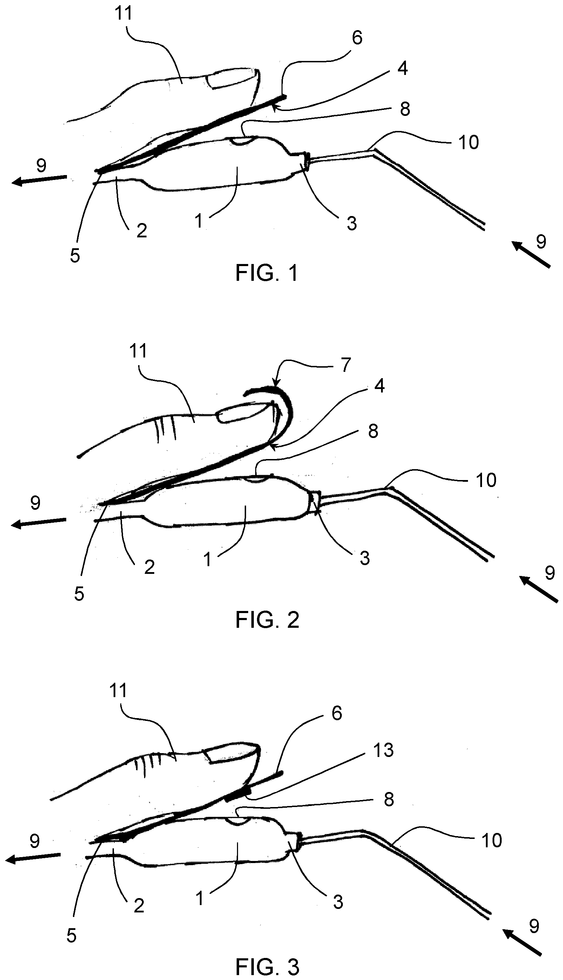

The suction tube and/or the suction pipe may be removably attached to the suction control handle, such that e.g. the suction pipe can be discarded after each use on a patient. Alternatively, the suction control handle and the suction pipe may be integrated as one unit. Embodiments, wherein the handle and the suction pipe form one unit are typically used multiple times, since they are often suitable for being thoroughly rinsed after use. Fig. 1 shows a suction control handle, wherein a suction pipe is attached. In general, the various parts of the medical suction device may comprise any type of material suitable for the application. Hence, the suction control handle and/or the suction pipe and/or the suction tube(s) and/or the cover element may comprise (or be made entirely from) one or more materials such as metal, plastic, silicone, rubber, polymer, and/or combinations thereof.

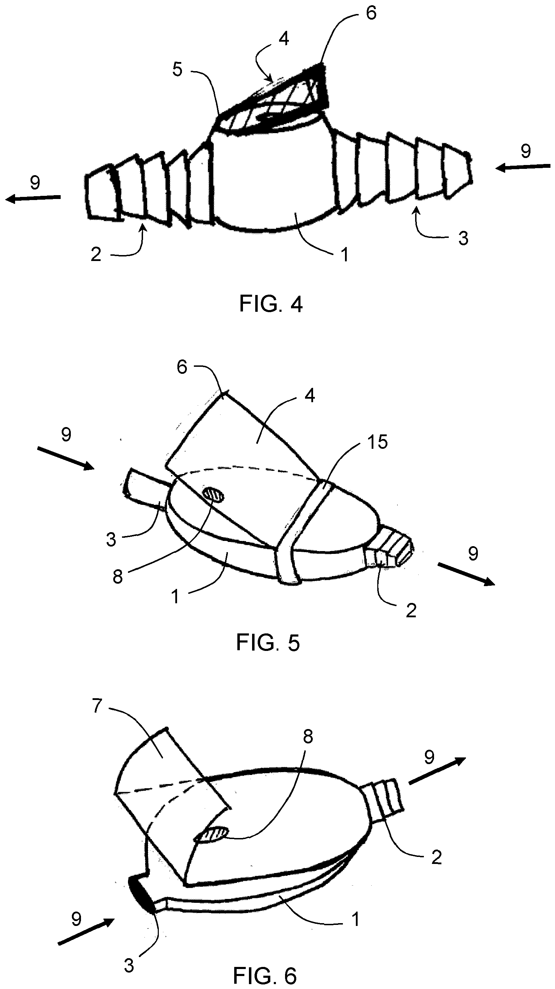

As explained above, the suction pipe may be removably attached to the suction control handle or it may form an integral part of the suction control handle. In another embodiment, the suction control handle comprises two ends, wherein both the proximal end and the distal end are configured for attaching a suction tube. According to this embodiment, the proximal end of the suction control handle is configured to connect to a first suction tube, and the distal end of the suction control handle is configured to connect to a second suction tube. This embodiment is shown in Fig. 4. Accordingly, the different embodiments of the suction control handle comprise different connection functionalities at the two ends (i.e. they are configured for connecting to various types of medical equipment); however, the suction control handles have many features in common across the different embodiments. The suction control handle preferably comprises an inlet for receiving an airflow, an outlet for fluidly connecting the control handle to a suction tube or similar, and a channel fluidly connecting the inlet and the outlet. Preferably, the channel is suitable for directing the airflow through the suction control handle from the inlet to the outlet. The suction control handle further comprises an aeration hole fluidly connected to said channel, such that the aeration hole is suitable for regulating the airflow through the suction control handle during use. Additionally, the suction control handle comprises a cover element formed such that, in use, the airflow through the suction control handle can be gradually adjusted by covering the aeration hole, wholly or partly, by the cover element.

According to one embodiment, the cover element is a bendable flap comprising a fixed end attached to the suction control handle and a free end having at least one degree of freedom. In this embodiment, the cover element is configured to cover the aeration hole upon applied pressure to the free end, e.g. by a finger, said pressure being directed towards the aeration hole. Preferably, the cover element is further configured such that the free end of the cover element is configured to return to an equilibrium position above said aeration hole upon release of said pressure. This may be achieved by making the cover element in a flexible material having a certain elasticity / spring constant. Thereby, the cover element has a restoring force when the pressure on top of the cover element is released, wherein said restoring force is able to lift the free end of the cover element to an equilibrium position above the aeration hole. The fixed end of the cover element may be attached to a proximal end of the suction control handle, wherein a distal section of the cover element is configured to cover the aeration hole upon applied pressure on top of the cover element.

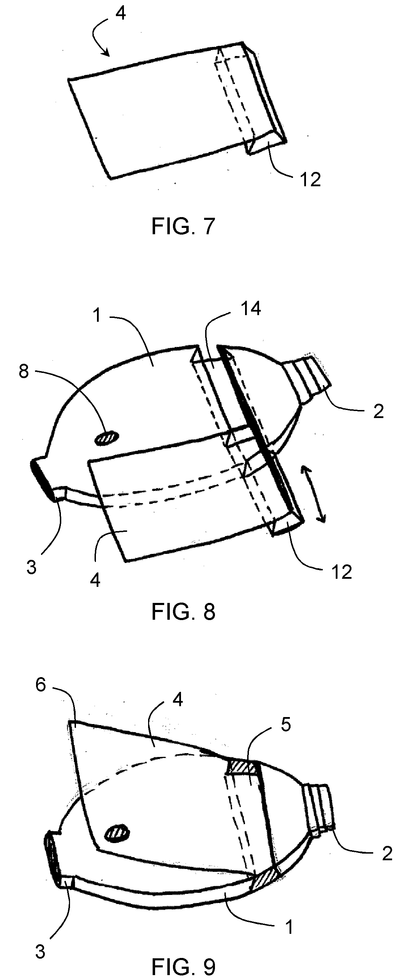

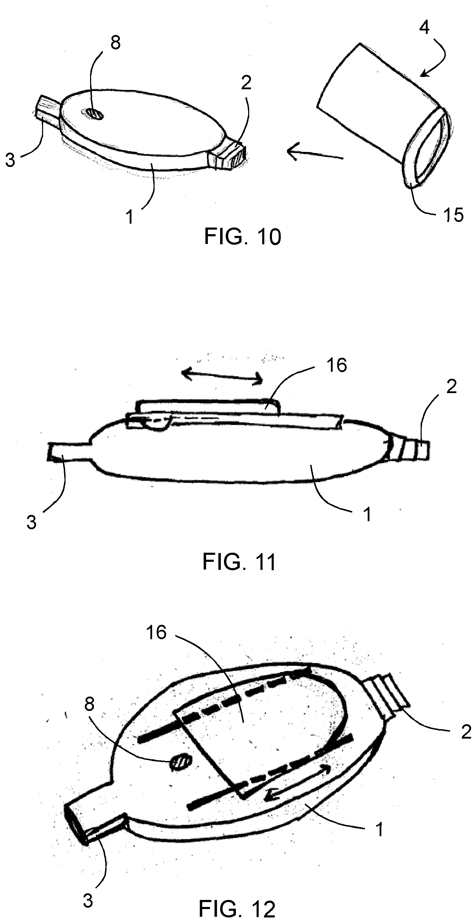

The cover element may be configured to be removably attached to the suction control handle, or it may form an integral part of the suction control handle. Hence, the cover element may be replaceable. The cover element may be attached to the suction control handle by any suitable attachment means, e.g. using an adhesive or an elastic band. Fig. 10 shows a cover element comprising an elastic band configured for attaching the cover element to the suction control handle. Alternatively, the cover element may be permanently attached to the suction control handle. In case the cover element is configured for removable attachment, it may comprise a first protrusion configured to mechanically engage with a groove in the suction control handle, such that the cover element can be removably attached to the control handle by sliding the first protrusion into said groove. This is exemplified in Fig. 8. Preferably, the first protrusion of the cover element stays in position in the groove during use because of mechanical friction. Accordingly, the suction control handle may comprise a groove configured to receive and secure a first protrusion of the cover element. The first protrusion of the cover element may preferably be positioned near the fixed end of the cover element. Additionally or alternatively, the cover element may comprise a second protrusion attached to the cover element, said protrusion configured to mechanically engage with the aeration hole, such that the second protrusion can be wholly or partly inserted in the aeration hole. In case the cover element comprises the second protrusion, said second protrusion is preferably located near the free end of the cover element as indicated in Fig. 3. In case the cover element is a bendable flap, the flap is preferably adapted to the shape of the suction control handle, such that it approximately conforms to the surface of the control handle. Hence, the cover element may be convex and/or concave.

According to the invention as claimed, the cover element comprises an absorbent pad, said absorbent pad comprising an absorbent material for absorbing liquids or body fluids. The absorbent pad may be a foam pad, a hydrogel pad or similar. The absorbent pad has the purpose of absorbing at least some of the unwanted substances exiting through the aeration hole. The absorbent pad is preferably placed underneath the cover element in the area that contacts the aeration hole during use. In the embodiment wherein the cover element is a bendable flap comprising a fixed end and a free end, the absorbent pad is preferably placed near the free end on the lower side of the flap. The cover element may be made of any suitable material such as silicone, metal, plastic, or combinations thereof.

In one embodiment, the cover element comprises a pocket configured to receive a fingertip, such as the tip of a thumb, of a person operating the medical suction device. Preferably, the pocket is configured such that it allows, during use, a person to elevate the free end of the cover element by lifting the pocket. Preferably, the pocket is positioned near or at the free end of the cover element. In one embodiment, the free end is curved backwards to form the pocket.

According to another embodiment, the cover element comprises a slidable cover plate configured to cover the aeration hole upon sliding the cover plate across the aeration hole. This embodiment is shown in figures 11-12. The cover plate is preferably integrated in the suction control handle and configured to have one degree of freedom, such that it may slide back and forth in the longitudinal direction of the control handle as indicated by the double arrow in Fig. 12.

All of the embodiments of the cover element may be used in combination with any of the embodiments of the suction control handle.

### Reference numerals

- 1.: Suction control handle
- 2.: Proximal end of the suction control handle
- 3.: Distal end of the suction control handle
- 4.: Cover element
- 5.: Fixed end of cover element
- 6.: Free end of cover element
- 7.: Pocket
- 8.: Aeration hole
- 9.: Direction of airflow
- 10.: Suction pipe
- 11.: Finger
- 12.: First protrusion
- 13.: Second protrusion
- 14.: Groove
- 15.: Elastic band
- 16.: Cover plate

## Claims

1. A suction control handle (1) for a medical suction device, said medical suction device being configured for creating a vacuum or connected to a vacuum supply, such that an airflow is present through the suction control (1) handle during use, the suction control handle (1) comprising:
- an aeration hole (8) for regulating the airflow through the suction control handle (1) during use; and
- a cover element (4) attached to the suction control handle (1), and formed such that, in use, the airflow through the suction control handle (1) can be gradually adjusted by covering the aeration hole (8), wholly or partly, by the cover element (4),
the suction control handle **characterized in that** the cover element (4) comprises an absorbent pad, said absorbent pad comprising an absorbent material for absorbing liquids or body fluids exiting through the aeration hole (8).

2. The suction control handle according to claim 1, wherein the cover element (4) is a bendable flap comprising a fixed end (5) attached to the suction control handle (1) and a free end (6) having at least one degree of freedom.

3. The suction control handle according to claim 2, wherein the cover element (4) is configured to cover the aeration hole (8) upon applied pressure to the free end (6), e.g. by a finger, said pressure being directed towards the aeration hole (8), and/or
wherein the cover element (4) conforms to a surface of the suction control handle (1), preferably the cover element is convex or concave.

4. The suction control handle according to any of the claims 2-3, wherein the free end (6) of the cover element (4) is configured to return to an equilibrium position above said aeration hole (8) upon release of said pressure.

5. The suction control handle according to any of the claims 2-4, wherein the fixed end (5) of the cover element (4) is attached to a proximal end of the suction control handle (1), and wherein a distal section of the cover element (4) is configured to cover the aeration hole (8) upon applied pressure on top of the cover element (4).

6. The suction control handle according to any of the preceding claims, wherein the cover element (4) is removably attached to the suction control handle (1).

7. The suction control handle according to any of the preceding claims, wherein the cover element (4) comprises an elastic band (15) configured to attach to the suction control handle (1).

8. The suction control handle according to any of the preceding claims, comprising a groove (14) configured to receive and secure the fixed end (5) of the cover element (4).

9. The suction control handle according to claim 8, wherein the cover element (4) comprises a first protrusion (12) configured to mechanically engage with the groove (14), such that the cover element (4) can be removably attached to the suction control handle (1) by sliding the first protrusion (12) into said groove (14).

10. The suction control handle according to any of the preceding claims, wherein the cover element (4) comprises a second protrusion (13) configured to mechanically engage with the aeration hole (8), such that the second protrusion (13) can be wholly or partly inserted in the aeration hole (8).

11. The suction control handle according to any of the preceding claims, wherein the cover element (4) comprises a pocket (7) configured to receive a fingertip, such as the tip of a thumb, of a person operating the medical suction device, wherein said pocket (7) allows, during use, a person to elevate the free end (6) of the cover element (4).

12. The suction control handle according claim 11, wherein the free end (6) is curved backwards to form the pocket (7).

13. The suction control handle according to any of the preceding claims, wherein the absorbent pad is foam pad, or a hydrogel pad.

14. The suction control handle according to any of the preceding claims, wherein the absorbent pad is located underneath the cover element (4) in the area that contacts the aeration hole (8) during use.

15. The suction control handle according to any of the preceding claims, wherein the cover element (4) comprises a slidable cover plate (16) configured to cover the aeration hole (8) upon sliding the cover plate (16) across the aeration hole (8).

## Patentansprüche

1. Saugsteuergriff (1) für eine medizinische Saugvorrichtung, wobei die medizinische Saugvorrichtung derart zum Erzeugen eines Vakuums ausgelegt oder mit einer Vakuumversorgung verbunden ist, dass während der Verwendung ein Luftstrom durch den Saugsteuergriff (1) vorhanden ist, wobei der Saugsteuergriff (1) Folgendes umfasst:
- ein Belüftungsloch (8) zur Regulierung des Luftstroms durch den Saugsteuergriff (1) während der Verwendung; und
- ein Abdeckelement (4), das an dem Saugsteuergriff (1) angebracht ist und derart ausgebildet ist, dass der Luftstrom durch den Saugsteuergriff (1) bei der Verwendung durch vollständiges oder partielles Abdecken des Belüftungslochs (8) durch das Abdeckelement (4) allmählich eingestellt werden kann, wobei der Saugsteuergriff **dadurch gekennzeichnet ist, dass** das Abdeckelement (4) ein absorbierendes Kissen umfasst, wobei das absorbierende Kissen ein absorbierendes Material zum Absorbieren von durch das Belüftungsloch (8) austretende Flüssigkeiten oder Körperfluide umfasst.

2. Saugsteuergriff nach Anspruch 1, wobei das Abdeckelement (4) eine biegbare Klappe ist, die ein an dem Saugsteuergriff (1) angebrachtes festes Ende (5) und ein mindestens einen Freiheitsgrad aufweisendes freies Ende (6) umfasst.

3. Saugsteuergriff nach Anspruch 2, wobei das Abdeckelement (4) zum Abdecken des Belüftungslochs (8) bei auf das freie Ende (6) ausgeübtem Druck, z. B. durch einen Finger, ausgelegt ist, wobei der Druck in Richtung des Belüftungslochs (8) gerichtet ist, und/oder
wobei das Abdeckelement (4) an eine Fläche des Saugsteuergriffs (1) angepasst ist, wobei das Abdeckelement vorzugsweise konvex oder konkav ist.

4. Saugsteuergriff nach einem der Ansprüche 2-3, wobei das freie Ende (6) des Abdeckelements (4) bei Entlastung des Drucks zum Zurückkehren in eine Gleichgewichtsposition über dem Belüftungsloch (8) ausgelegt ist.

5. Saugsteuergriff nach einem der Ansprüche 2-4, wobei das feste Ende (5) des Abdeckelements (4) an einem proximalen Ende des Saugsteuergriffs (1) angebracht ist und wobei ein distaler Abschnitt des Abdeckelements (4) zum Abdecken des Belüftungslochs (8) bei angelegtem Druck auf die Oberseite des Abdeckelements (4) ausgelegt ist.

6. Saugsteuergriff nach einem der vorhergehenden Ansprüche, wobei das Abdeckelement (4) lösbar an dem Saugsteuergriff (1) angebracht ist.

7. Saugsteuergriff nach einem der vorhergehenden Ansprüche, wobei das Abdeckelement (4) ein elastisches Band (15) umfasst, das zum Anbringen an dem Saugsteuergriff (1) ausgelegt ist.

8. Saugsteuergriff nach einem der vorhergehenden Ansprüche, umfassend eine Nut (14), die zum Aufnehmen und Sichern des festen Endes (5) des Abdeckelements (4) ausgelegt ist.

9. Saugsteuergriff nach Anspruch 8, wobei das Abdeckelement (4) einen ersten Vorsprung (12) umfasst, der derart zum mechanischen Eingreifen in die Nut (14) ausgelegt ist, dass das Abdeckelement (4) durch Schieben des ersten Vorsprungs (12) in die Nut (14) lösbar an dem Saugsteuergriff (1) angebracht werden kann.

10. Saugsteuergriff nach einem der vorhergehenden Ansprüche, wobei das Abdeckelement (4) einen zweiten Vorsprung (13) umfasst, der derart zum mechanischen Eingreifen in das Belüftungsloch (8) ausgelegt ist, dass der zweite Vorsprung (13) ganz oder teilweise in das Belüftungsloch (8) eingesetzt werden kann.

11. Saugsteuergriff nach einem der vorhergehenden Ansprüche, wobei das Abdeckelement (4) eine Tasche (7) umfasst, die zum Aufnehmen einer Fingerspitze, wie der Spitze eines Daumens, einer die medizinische Saugvorrichtung bedienenden Person ausgelegt ist, wobei die Tasche (7) einer Person während der Verwendung ein Anheben des freien Endes (6) des Abdeckelements (4) ermöglicht.

12. Saugsteuergriff nach Anspruch 11, wobei das freie Ende (6) unter Bilden der Tasche (7) nach hinten gekrümmt ist.

13. Saugsteuergriff nach einem der vorhergehenden Ansprüche, wobei das absorbierende Kissen ein Schaumstoffkissen oder ein Hydrogelkissen ist.

14. Saugsteuergriff nach einem der vorhergehenden Ansprüche, wobei sich das absorbierende Kissen unter dem Abdeckelement (4) in dem Bereich befindet, der das Belüftungsloch (8) während der Verwendung berührt.

15. Saugsteuergriff nach einem der vorhergehenden Ansprüche, wobei das Abdeckelement (4) eine verschiebbare Abdeckplatte (16) umfasst, die beim Schieben der Abdeckplatte (16) über das Belüftungsloch (8) zum Abdecken des Belüftungslochs (8) ausgelegt ist.

## Revendications

1. Poignée de commande d'aspiration (1) pour un dispositif d'aspiration médical, ledit dispositif d'aspiration médical étant configuré pour créer un vide ou relié à une alimentation en vide, de sorte qu'un flux d'air est présent à travers la poignée de commande d'aspiration (1) pendant l'utilisation, la poignée de commande d'aspiration (1) comprenant :
- un trou d'aération (8) pour réguler le flux d'air à travers la poignée de commande d'aspiration (1) pendant l'utilisation ; et
- un élément de couvercle (4) fixé à la poignée de commande d'aspiration (1), et formé de telle sorte que, lors de l'utilisation, le flux d'air à travers la poignée de commande d'aspiration (1) peut être progressivement ajusté en recouvrant le trou d'aération (8), en totalité ou en partie, avec l'élément de couvercle (4),
la poignée de commande d'aspiration étant **caractérisée en ce que** l'élément de couvercle (4) comprend un tampon absorbant, ledit tampon absorbant comprenant un matériau absorbant pour absorber des liquides ou des fluides corporels sortant par le trou d'aération (8).

2. Poignée de commande d'aspiration selon la revendication 1, dans laquelle l'élément de couvercle (4) est un volet pliable comprenant une extrémité fixe (5) fixée à la poignée de commande d'aspiration (1) et une extrémité libre (6) ayant au moins un degré de liberté.

3. Poignée de commande d'aspiration selon la revendication 2, dans laquelle l'élément de couvercle (4) est configuré pour recouvrir le trou d'aération (8) lors d'une pression appliquée sur l'extrémité libre (6), par exemple par un doigt, ladite pression étant dirigée vers le trou d'aération (8), et/ou dans laquelle l'élément de couvercle (4) épouse une surface de la poignée de commande d'aspiration (1), de préférence l'élément de couvercle est convexe ou concave.

4. Poignée de commande d'aspiration selon l'une quelconque des revendications 2 et 3, dans laquelle l'extrémité libre (6) de l'élément de couvercle (4) est configurée pour revenir en position d'équilibre au-dessus dudit trou d'aération (8) lors de la libération de ladite pression.

5. Poignée de commande d'aspiration selon l'une quelconque des revendications 2 à 4, dans laquelle l'extrémité fixe (5) de l'élément de couvercle (4) est fixée à une extrémité proximale de la poignée de commande d'aspiration (1), et dans laquelle une section distale de l'élément de couvercle (4) est configurée pour recouvrir le trou d'aération (8) lors d'une pression appliquée sur le dessus de l'élément de couvercle (4).

6. Poignée de commande d'aspiration selon l'une quelconque des revendications précédentes, dans laquelle l'élément de couvercle (4) est fixé de manière amovible à la poignée de commande d'aspiration (1).

7. Poignée de commande d'aspiration selon l'une quelconque des revendications précédentes, dans laquelle l'élément de couvercle (4) comprend une bande élastique (15) configurée pour se fixer à la poignée de commande d'aspiration (1).

8. Poignée de commande d'aspiration selon l'une quelconque des revendications précédentes, comprenant une rainure (14) configurée pour recevoir et fixer l'extrémité fixe (5) de l'élément de couvercle (4).

9. Poignée de commande d'aspiration selon la revendication 8, dans laquelle l'élément de couvercle (4) comprend une première saillie (12) configurée pour venir en prise mécaniquement avec la rainure (14), de sorte que l'élément de couvercle (4) peut être fixé de manière amovible à la poignée de commande d'aspiration (1) par coulissement de la première saillie (12) dans ladite rainure (14).

10. Poignée de commande d'aspiration selon l'une quelconque des revendications précédentes, dans laquelle l'élément de couvercle (4) comprend une seconde saillie (13) configurée pour venir en prise mécaniquement avec le trou d'aération (8), de sorte que la seconde saillie (13) peut être insérée en totalité ou en partie dans le trou d'aération (8).

11. Poignée de commande d'aspiration selon l'une quelconque des revendications précédentes, dans laquelle l'élément de couvercle (4) comprend une poche (7) configurée pour recevoir le bout d'un doigt, tel que le bout d'un pouce, d'une personne actionnant le dispositif d'aspiration médical, dans laquelle ladite poche (7) permet, lors de l'utilisation, à une personne de soulever l'extrémité libre (6) de l'élément de couvercle (4).

12. Poignée de commande d'aspiration selon la revendication 11, dans laquelle l'extrémité libre (6) est recourbée vers l'arrière pour former la poche (7).

13. Poignée de commande d'aspiration selon l'une quelconque des revendications précédentes, dans laquelle le tampon absorbant est un tampon de mousse ou un tampon d'hydrogel.

14. Poignée de commande d'aspiration selon l'une quelconque des revendications précédentes, dans laquelle le tampon absorbant est situé sous l'élément de couvercle (4) dans la zone en contact avec le trou d'aération (8) pendant l'utilisation.

15. Poignée de commande d'aspiration selon l'une quelconque des revendications précédentes, dans laquelle l'élément de couvercle (4) comprend une plaque de couvercle coulissante (16) configurée pour recouvrir le trou d'aération (8) lors du coulissement de la plaque de couvercle (16) à travers le trou d'aération (8).
